# EUROPÄISCHE PATENTSCHRIFT

(11) **EP 3 368 510 B1**
(45) Veröffentlichungstag und Bekanntmachung des Hinweises auf die Patenterteilung: **19.02.2020**
(21) Anmeldenummer: 16785143.5
(22) Anmeldetag: 21.10.2016
(51) Int. Cl.: C07C 315/02, C07C 319/14, C07C 319/20, C07C 253/14, C07C 255/50, C07C 25/13, C07C 323/62

(54) **VERFAHREN ZUR HERSTELLUNG VON 2-ALKYL-4-TRIFLUORMETHYL-3-ALKYLSULFONYLBENZOESÄUREN**
METHOD FOR THE PREPARATION OF 2-ALKYL-4-TRIFLUORMETHYL-3-ALKYLSULFONYL BENZOIC ACIDS
PROCÉDÉ DE FABRICATION D'ACIDES 2-ALKYL-4-TRIFLUORMÉTHYLE-3-ALKYLSULFONYLE-BENZOÏQUES

(30) Priorität: 26.10.2015 EP 15191494
(43) Veröffentlichungstag der Anmeldung: 05.09.2018
(73) Patentinhaber: Bayer CropScience Aktiengesellschaft, 40789 Monheim am Rhein (DE)
(72) Erfinder: FUNKE, Christian, 42799 Leichlingen (DE); HIMMLER, Thomas, 51519 Odenthal (DE); PAZENOK, Sergii, 42699 Solingen (DE); SCHOTES, Christoph, 40223 Düsseldorf (DE); BELLER, Matthias, 18211 Nienhagen (DE); SCHAREINA, Thomas, 18195 Cammin (DE); ZAPF, Alexander, 18055 Rostock (DE)
(74) Vertreter: BIP Patents
(86) Internationale Anmeldenummer: PCT/EP2016/075361
(87) Internationale Veröffentlichungsnummer: WO 2017/072038

(56) Entgegenhaltungen:
- WO-A1-2005/044007
- WO-A1-2008/125214
- CN-B- 102 010 351
- GB-A- 2 194 235
- SAKAKIBARA Y ET AL: "THE CYANATION OF AROMATIC HALIDES CATALYZED BY NICKEL(0) COMPLEXES GENERATED IN SITU I. GENERAL SCOPE AND LIMITATION", BULLETIN OF THE CHEMICAL SOCIETY OF JAPAN, CHEMICAL SOCIETY OF JAPAN, TOKYO, JP, Bd. 61, 1. Januar 1988 (1988-01-01), Seiten 1985-1990, XP001037638, ISSN: 0009-2673 in der Anmeldung erwähnt

## Beschreibung

Die Erfindung betrifft ein Verfahren zur Herstellung von 2-Alkyl-4-trifluormethyl-3-alkylsulfonylbenzoesäuren, die als Intermediate zur Herstellung von agrochemisch wirksamen Stoffen von Nutzen sind.

Aus mehreren Schriften sind agrochemisch wirksame Stoffe bekannt, zu deren Herstellung 2-Alkyl-4-trifluormethyl-3-alkylsulfonylbenzoesäuren benötigt werden. So sind aus WO 2008/125214 A1 herbizid wirksame 4-(4-Trifluormethyl-3-thiobenzoyl)-pyrazole bekannt. Aus WO 2012/126932 A1 sind herbizid wirksame N-(1,3,4-oxadiazol-2-yl)arylcarboxamide, unter anderem auch solche mit einem ähnlichen Suibstitutionsmuster im Phenylring wie die in WO 2008/125214 A1 offenbarten Verbindungen, bekannt.

WO 2008/125214 A1 offenbart auch ein Verfahren zur Herstellung der Verbindung 2-Methyl-4-trifluormethyl-3-methylsulfonylbenzoesäure. Bei diesem Verfahren wird 3-Fluor-2-methyl-4-trifluormethylbenzoesäure mit Natriumhydrid und Natriumthiomethylat zur 2-Methyl-3-methylthio-4-trifluormethylbenzoesäure umgesetzt, die anschließend zur 2-Methyl-3-methylsulfonyl-4-trifluormethylbenzoesäure oxidiert wird.
Nachteilig diese Verfahrens ist die Verwendung von 3-Fluor-2-methyl-4-trifluormethyl-benzoesäure, die auf Grund des Fluorsubstituenten am Phenylring in aufwendiger Art und Weise hergestellt werden muss, sowie die Einführung der Methylgruppe durch Metallierung der 3-Fluor-2-methyl-4-trifluormethyl-benzoesäure mit mindestens 2 Moläquivalenten Butyllithium bei tiefer Temperatur, gefolgt von einer Umsetzung mit dem toxischen Methyliodid. Dieses Verfahren ist aufwendig und wegen der nur geringen Ausbeute bei der Einführung der Methylgruppe (50,7% der Theorie) zudem unökonomisch.

Ebenfalls bekannt sind Verfahren zur Herstellung von substituierten Benzoesäuren durch übergangsmetallkatalysierte Cyanierungen von Chloraromaten und anschließender Verseifung der Cyanogruppe zur Säuregruppe. Insbesondere Palladium- und Nickelverbindungen werden häufig als Katalysatoren verwendet, wobei Nickelkatalysatoren wegen des deutlich geringeren Preises aus ökonomischer Sicht zu bevorzugen sind. Die nickelkatalysierte Cyanierung von Arylhalogeniden mit Natriumcyanid in Gegenwart von Ni(PPh₃)₃ oder [Aryl-Ni(PPh₃)₂Cl] ist bekannt, J. Organomet. Chem., 54, 1973, C57. Ebenfalls bekannt ist die Cyanierung von trifluormethyl-substituierten Halogenaromaten. So wird beispielsweise in Bull. Chem. Soc. Jpn. 61 (1988)1985-1990 die Reaktion von meta- oder para-Chlorbenzotrifluorid mit Kaliumcyanid in Gegenwart von NiBr₂(PPh₃)₂ und metallischem Zink zu meta- oder para-Trifluormethylbenzonitril beschrieben. Als weitere Liganden außer Triphenylphosphin (PPh₃) wurden hier Tri(o-tolyl)phosphin, 1,2-Bis(diphenylphosphino) ethan (dppe) und 1,1'-Bis(diphenylphosphino)ferrocen (dppf) verwendet, wobei sich außer dem PPh₃ nur das dppf als wirksam erwies. CN 102010351 beschreibt eine Methode zur Herstellung von 2-Fluor-4-cyano-trifluormethylbenzol durch Cyanierung von 2,4-Dichlor-benzotrifluorid mit Natrium- oder Kaliumcyanid in Gegenwart von NiBr₂(PPh₃)₂. Die Offenbarung der vorstehend genannten Schriften zeigt, dass die katalytische Cyanierung von Arylhalogeniden jedoch oft mit nicht zufriedenstellenden Ausbeuten verläuft.

Aufgabe der vorliegenden Erfindung ist die Bereitstellung eines Verfahrens zur Herstellung von 2-Alkyl-4-trifluormethyl-3-alkylsulfonylbenzoesäuren, welches die Nachteile der aus dem Stand der Technik bekannten Verfahren überwindet.

Es wurde nun gefunden, dass 2-Alkyl-4-trifluormethyl-3-alkylsulfonylbenzoesäuren kostengünstig und in hohen Ausbeuten hergestellt werden können durch Cyanierung von 1,3-Dichlor-2-alkyl-4-trifluormethylbenzolen in Gegenwart einer Nickelverbindung, eines Phosphinliganden und eines weiteren Metalles, Thiolierung des erhaltenen Benzonitrils, Verseifung der Nitrilgruppe und anschließender Oxidation der Thiogruppe.

Ein Gegenstand vorliegender Erfindung ist somit ein Verfahren zur Herstellung von 2-Alkyl-4-trifluormethyl-3-alkylsulfonylbenzoesäuren der allgemeinen Formel (I), dadurch gekennzeichnet, dass
a) in einem ersten Schritt ein 1,3-Dichlor-2-alkyl-4-trifluormethylbenzol mit einer Cyanidquelle in Gegenwart einer Nickelverbindung, eines Phosphinliganden und eines weiteren Metalles zu einem Benzonitril umgesetzt wird,
b) in einem zweiten Schritt das Benzonitril mit einem Thiolat in Anwesenheit eines Phasentransferkatalysators zum entsprechenden Thioether umgesetzt wird,
c) in einem dritten Schritt die Nitrilgruppe zur Carboxylgruppe verseift wird,
d) in einem vierten Schritt die Thiogruppe mit Wasserstoffperoxid oxidiert wird, gegebenenfalls in Gegenwart eines Oxidationskatalysators, 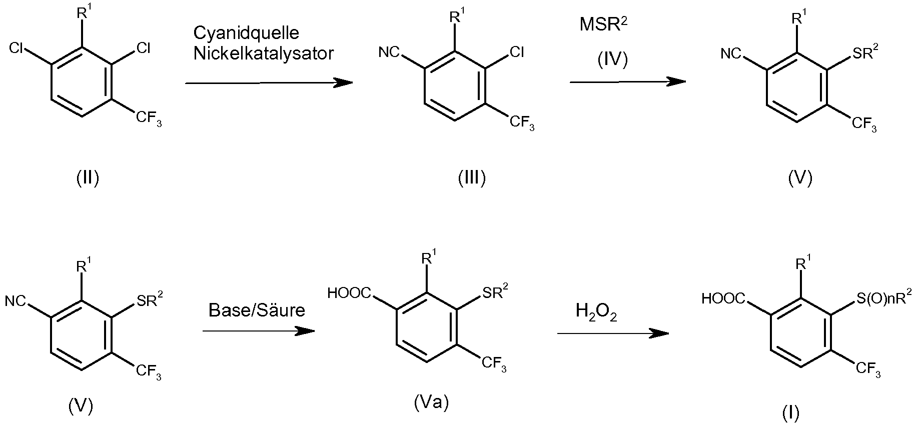 und
e) worin die Substituenten wie nachfolgend definiert sind:
   - R¹ und R²: bedeuten unabhängig voneinander C₁-C₄-Alkyl oder durch s Reste aus der Gruppe bestehend aus Chlor, Fluor, Methoxy und Ethoxy substituiertes Phenyl,
   - M: bedeutet Lithium, Natrium oder Kalium,
   - n: bedeutet 1 oder 2,
   - s: bedeutet 1, 2 oder 3.

Die zur Durchführung des erfindungsgemäßen Verfahrens notwendigen Verbindungen der Formel (II) sind nach allgemeinen dem Fachmann bekannten Methoden zugänglich. So beschreibt beispielsweise WO 2005/044007 A1 die Herstellung von analogen Verbindungen der Formel (II), worin R¹ für gegebenenfalls substituiertes Phenyl steht. Verbindungen der Formel (II), worin R¹ für C₁-C₄-Alkyl steht, können beispielsweise durch Deprotonierung von 2,4-Dichlorbenzotrifluorid mit Lithiumdiisopropylamid und anschließender Alkylierung mit einem Dialkylsulfat hergestellt werden. GB 2 194 235 A beschreibt ein Verfahren zur Herstellung von 2,6-Dichlor-3-trifluormethyltoluolen.

Wesentliche Vorteile des erfindungsgemäßen Verfahrens sind die hohen Ausbeuten, die Verwendung preisgünstiger Reagenzien und die geringen Mengen an Abfallstoffen.

In den Formeln (I), (II), (III), (IV) und (V) können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl.

In dem erfindungsgemäßen Verfahren bedeuten R¹ und R² jeweils Methyl.

Im ersten Schritt des erfindungsgemäßen Verfahrens können als Cyanidquellen anorganische Cyanide, wie Lithiumcyanid, Natriumcyanid, Kaliumcyanid, Magnesiumcyanid, Calciumcyanid, Zinkcyanid oder Kaliumhexacyanoferrat(II), und organische Cyanoverbindungen, wie Cyanhydrine, verwendet werden. Bevorzugt sind Natriumcyanid, Kaliumcyanid, Zinkcyanid, Kaliumhexacyanoferrat(II) und Acetoncyanhydrin. Besonders bevorzugt sind Natriumcyanid und Kaliumcyanid. Die Cyanidquellen können als Reinstoff in einer Portion oder portionsweise zugegeben werden. Es ist auch möglich, gegebenenfalls eine Lösung der Cyanidquelle zuzudosieren, beispielsweise als 30%ige Lösung von Natriumcyanid in Wasser. Bevorzugt sind Reaktionen, bei denen 1 bis 10 Molprozent Wasser bezogen auf Verbindung der Formel (II) im Reaktionsgemisch sind. Besonders bevorzugt sind 1,5 bis 5 Molprozent.

Die Cyanidquelle wird in einem Molverhältnis von 0,7:1 bis 2:1, bezogen auf die Verbindung der allgemeinen Formel (II) eingesetzt. Bevorzugt wird sie in einer Menge von 1,5:1 bis 1:1, besonders bevorzugt von 1,2:1 bis 1,1:1 eingesetzt.

Die Reaktion des ersten Schritts des erfindungsgemäßen Verfahrens wird in der Regel in einem Lösungsmittel durchgeführt. Bevorzugt sind Acetonitril, Aceton, Methylethylketon, Tetrahydrofuran (THF), Butyronitril und Isopropanol. Besonders bevorzugt sind Acetonitril, Aceton, THF und Methylethylketon.

Im ersten Schritt des erfindungsgemäßen Verfahrens können als Nickelverbindung Nickel(0)- und Nickel(II)-Verbindungen wie Bis(1,5-cyclooctadien)nickel(0), Bis(cyclopentadienyl)nickel, Methallylnickelchlorid-Dimer, Nickel(II)chlorid, Nickel(II)-bromid, Nickel(II)acetat, Nickel(II)acetylacetonat, Nickel(II)nitrat, in Kombination mit einem Phosphinliganden eingesetzt werden. Bevorzugt sind Nickel(II)chlorid und Nickel(II)bromid, besonders bevorzugt ist Nickel(II)bromid. Die katalytisch wirksamen Nickelverbindungen werden in Mengen von 0,1 bis 20 Molprozent, bezogen auf die Verbindung der allgemeinen Formel (II) eingesetzt. Bevorzugt sind 0,5 bis 5 Molprozent, besonders bevorzugt 1 bis 2 Molprozent.

Im ersten Schritt des erfindungsgemäßen Verfahrens können als Phosphinliganden die nachfolgend genannten verwendet werden: 1,1'-Bis(diphenylphosphino)ferrocen (dppf), rac-2,2'-Bis(diphenylphosphino)-1,1'-binaphthyl (BINAP), (R)-(+)-2,2'-Bis(diphenylphosphino)-1,1'-binaphthyl, (S)-(-)-2,2'-Bis(diphenylphosphino)-1,1'-binaphthyl, rac-5,5'-Bis(diphenylphosphino)-4,4'-bi-1,3-benzodioxol, 2,2'-Bis(diphenylphosphino)-1,1'-biphenyl, rac-2,2'-Bis(diphenylphosphino)-5,5',6,6',7,7',8,8'-octahydro-1,1'-binaphthyl, rac-6,6'-Bis(diphenylphosphino)-2,2',3,3'-tetrahydro-5,5'-bi-1,4-benzodioxin, rac-2,2'-Bis(di-p-tolylphosphino)-1,1'-binaphthyl, Bis(2-diphenylphosphinophenyl)ether (dpephos), Triphenylphosphin, Trifufurylphosphin, Tri(o-tolyl)phosphin, Tri(p-tolyl)phosphine, P(tBu₃), Cataxcium A. Bevorzugt verwendet man 1,1'-Bis(diphenylphosphino)ferrocen (dppf), rac-2,2'-Bis(diphenylphosphino)-1,1'-binaphthyl(BINAP), (R)-(+)-2,2'-Bis(diphenylphosphino)-1,1'-binaphthyl, (S)-(-)-2,2'-Bis(diphenylphosphino)-1,1'-binaphthyl und Bis(2-diphenylphosphinophenyl)ether (dpephos); besonders bevorzugt sind 1,1'-Bis(diphenylphosphino)ferrocen (dppf), rac-2,2'-Bis(diphenylphosphino)-1,1'-binaphthyl (BINAP) und Bis(2-diphenylphosphinophenyl)ether (dpephos). Ganz besonders bevorzugt ist Bis(2-diphenylphosphinophenyl)ether (dpephos).

Die Phosphinliganden werden in einem Molverhältnis von 1:1 bis 5:1, bezogen auf die Nickelverbindung, eingesetzt. Bevorzugt werden sie in einem Molverhältnis von 1,5:1 bis 3:1, besonders bevorzugt von 2:1, eingesetzt.

Der Nickel(II) Komplex wird durch Zugabe eines Metalls wie Zink, Magnesium oder Mangan, vorzugsweise Zink, durch Reduktion aktiviert. Die Menge an Metall, bezogen auf die Verbindung der Formel (II), beträgt 1 bis 20 Molprozent. Bevorzugt werden 2 bis 10 Molprozent, besonders bevorzugt 6 Molprozent verwendet. Das Metall wird pulverförmig oder in Form feiner Späne eingesetzt. Das eingesetzte Nickel (II) Salz bildet mit dem Phosphinliganden nach Reduktion den eigentlich wirksamen Katalysator. Die Katalysatoren können separat hergestellt oder in situ gebildet werden. Falls während des Reaktionsverlaufs die Aktivität des Katalysators zu stark abnimmt, kann diese durch Zugabe von weiterem Reduktionsmittel (1-5 Molprozent) wieder erhöht werden.

Die Reaktion im ersten Schritt des erfindungsgemäßen Verfahrens wird in der Regel bei einer Temperatur von 25 bis 100° C, vorzugsweise 60 bis 90° C, besonders bevorzugt 70 bis 90° C, durchgeführt. Die Reaktion kann auch unter erhöhtem oder veringertem Druck durchgeführt werden.

Im zweiten Schritt des erfindungsgemäßen Verfahrens wird die Verbindung der allgemeinen Formel (IV) in einem Mengenverhältnis von 1:1 bis 2:1 Moläquivalenten bezogen auf die Verbindung der allgemeinen Formel (III) eingesetzt. Bevorzugt werden 1,1:1 bis 1,5:1 eingesetzt, besonders bevorzugt 1,3:1.

Die Verbindungen der allgemeinen Formel (IV) können sowohl in-situ also auch ex-situ aus den entsprechenden Thiolen und einer Base, wie Alkalimetallhydroxide, Erdalkalimetallhydroxide, Alkalimetallkarbonate, Alkalimetallacetate, Alkalimetallalkoholate und organischen Base, hergestellt werden. Geeignete Basen sind LiOH, NaOH, KOH, Ca(OH)₂, Na₂CO₃, K₂CO₃, Cs₂CO₃, NaOAc, KOAc, LiOAc, NaOMe, NaOEt, NaO-t-Bu,KO-t-Bu, Trialkylamine, Alkylpyridine, Phosphazene und 1,8-Diazabicyclo[5.4.0]undecen.

Die Reaktion des zweiten Schritts des erfindungsgemäßen Verfahrens kann auch in einem Lösungsmittel durchgeführt werden. Bevorzugt sind Methyl-t-butylether, Toluol, Chlorbenzol, o-Dichlorbenzol und Wasser.

Als Phasentransferkatalysator (PTC) werden Ammonium- oder Phosphonium-Salze, wie Tetrahexylammoniumchlorid, Tetrahexylammoniumbromid, Tetrahexylammoniumiodid, Tetraoctylammoniumchlorid, Tetraoctylammoniumbromid, Tetraoctylammoniumiodid, Aliquat HTA-1®, Aliquat 134®, Dimethyldidecylammoniumchlorid, Dimethyldodecylbenzylammoniumchlorid, Tributylhexadecylammoniumchlorid, Tributylhexadecylammoniumbromid, Tributyltetradecylphosphoniumchlorid und Tributyltetradecylphosphoniumbromid eingesetzt. Bevorzugt sind Aliquat 134® und Tributyltetradecylphosphoniumchlorid. Der Phasentransferkatalysator wird üblicherweise in einer Menge von 0,1 bis 10 Molprozent, bezogen auf die Verbindung der allgemeinen Formel (III) eingesetzt. Bevorzugt sind 1 bis 6 Molprozent, besonders bevorzugt 2 bis 4 Molprozent.

Die Reaktion im zweiten Schritt des erfindungsgemäßen Verfahrens wird in der Regel bei einer Temperatur von 10 bis 70° C, vorzugsweise 50 bis 60° C, durchgeführt. Die Verseifung der Nitrilgruppe zur Carboxylgruppe im dritten Schritt des erfindungsgemäßen Verfahrens erfolgt unter sauren Bedingungen in Gegenwart von mineralischen Säuren, wie H₂SO₄, HCl, HSO₃Cl, HF, HBr, HI, H₃PO₄ oder organischen Säuren wie CF₃COOH, p-Toluolsulfonsäure, Methansulfonsäure oder Trifluormethansulfonsäure, oder vorzugsweise unter basischen Bedingungen in Gegenwart von anorganischen Basen, wie LiOH, NaOH, KOH, Ca(OH)₂, Na₂CO₃, K₂CO₃, Cs₂CO₃, NaOMe, NaOEt, NaO-t-Bu, KO-t-Bu oder organischen Basen wie Trialkylaminen, Alkylpyridinen, Phosphazenen und 1,8-Diazabicyclo[5.4.0]undecen (DBU). Bevorzugt sind anorganische Basen wie NaOH, KOH, Na₂CO₃, K₂CO₃.

Die Reaktion im dritten Schritt des erfindungsgemäßen Verfahrens wird in der Regel bei einer Temperatur von 20 bis 200° C, vorzugsweise 70 bis 150° C, besonders bevorzugt 110 bis 130° C, durchgeführt.

Die Reaktion des dritten Schritts des erfindungsgemäßen Verfahrens wird in der Regel in einem Lösungsmittel durchgeführt. Geeignete Lösungsmittel sind Wasser, Alkohole wie Methanol, Ethanol, Isopropanol oder Butanol sowie aliphatische und aromatische Kohlenwasserstoffe, wie n-Hexan, Benzol oder Toluol, welche mit Heteroatomen wie Fluor oder Chlor substituiert sein können, wie Dichlormethan, Dichlorethan, Chlorbenzol oder Dichlorbenzol. Ebenfalls in Frage kommen Ether wie Diphenylether, Methyl-tert-butylether, Isopropylethylether, Dioxan, Diglyme, Dimethylglycol, Dimethoxyethan und THF; Amide wie Dimethylformamid und N-Methyl-2-pyrrolidon (NMP) und Mischungen solcher Lösungsmittel. Bevorzugt sind Wasser, und Alkohole wie Methanol, Ethanol, Isopropanol und Butanol. Besonders bevorzugt sind Methanol und n-Butanol.

Im vierten Schritt des erfindungsgemäßen Verfahrens wird die Thiogruppe der Verbindung (Va) mit Wasserstoffperoxid oxidiert, gegebenenfalls in Gegenwart eines Oxidationskatalysators Geeignete Oxydationskatalysatoren sind Na₂WO₄, Na₂MoO₄ sowie deren Hydrate, sowie Schwefelsäure in Kombination mit einer organischen Säure, wie Essigsäure, Ameisensäure oder Trifluoressigsäure.

Die Oxydationskatalysatoren werden in Mengen von 1 bis 20 Molprozent, bezogen auf die Verbindung der allgemeinen Formel (Va) eingesetzt. Bevorzugt sind 5 bis 15 Molprozent, besonders bevorzugt 10 Molprozent.

Wasserstoffperoxid wird in einer Menge von 2 bis 10, bevorzugt 3 bis 8, besonders bevorzugt 3,5 bis 5 Moläquivalenten, bezogen auf die Verbindung der allgemeinen Formel (Va), eingesetzt. Üblicherweise wird das Wasserstoffperoxid als 20-35 %-ige wässrige Lösung eingesetzt.

Die Reaktion im vierten Schritt des erfindungsgemäßen Verfahrens wird in der Regel bei einer Temperatur von 30 bis 100° C, vorzugsweise 40 bis 95° C, besonders bevorzugt 70 bis 95° C, durchgeführt.

Die Reaktion des vierten Schritts des erfindungsgemäßen Verfahrens wird in der Regel in einem Lösungsmittel durchgeführt. Geeignete Lösungsmittel sind Toluol, Chlorbenzol, Dichlorbenzol, Essigsäureethylester, Essigsäurebutylester, Essigsäure, Ameisensäure, Wasser sowie Gemische von Essigsäure oder Ameisensäure mit Wasser. Bevorzugt sind Toluol, Essigsäureethylester, Essigsäurebutylester, Essigsäure, Ameisensäure, Wasser sowie Gemische von Essigsäure oder Ameisensäure mit Wasser. Besonders bevorzugt sind Toluol, Essigsäurebutylester, Wasser, sowie Gemische von Essigsäure oder Ameisensäure mit Wasser.

Die Verbindungen der Formel (II), worin R¹ für bestimmte Substituenten steht, sind neu und eignen sich sehr gut als Ausgangsmaterialien für den ersten Schritt des erfindungsgemäßen Verfahrens. Offenbart sind somit die Verbindungen der Formel (IIa) 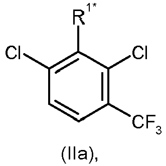 worin
- R^{1*}: bedeutet C₂-C₄-Alkyl oder durch s Reste aus der Gruppe bestehend aus Chlor, Fluor, Methoxy und Ethoxy substituiertes Phenyl,
- s: bedeutet 1, 2 oder 3.

Gegenstand der Erfindung sind Verbindungen der Formel (IIa), worin R^{1*} für Ethyl, n-Propyl oder Phenyl steht.

Verbindungen der Formel (III), worin R¹ für bestimmte Substituenten steht, sind neu und eignen sich sehr gut als Ausgangsmaterial für den zweiten Schritt des erfindungsgemäßen Verfahrens. Offenbart sind Verbindungen der Formel (IIIa) 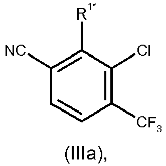 worin
- R^{1*}: bedeutet C₁-C₄-Alkyl oder durch s Reste aus der Gruppe bestehend aus Chlor, Fluor, Methoxy und Ethoxy substituiertes Phenyl,
- s: bedeutet 1, 2 oder 3.

Bevorzugt bedeutet R^{1*} in Formel (IIIa) Methyl, Ethyl, n-Propyl, iso-Propyl, n-Butyl oder Phenyl. Besonders bevorzugt bedeutet R^{1*} in Formel (IIIa) Methyl, Ethyl, n-Propyl oder Phenyl. Gegenstand der Erfindung sind Verbindungen der Formel (IIIa) worin R^{1*} in Formel (IIIa) für Methyl steht.

Verbindungen der Formel (V) sind neu und eignen sich sehr gut als Ausgangsmaterial für den dritten Schritt des erfindungsgemäßen Verfahrens. Offenbart sind somit Verbindungen der Formel (V), 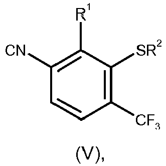 worin
- R¹ und R²: bedeuten unabhängig voneinander C₁-C₄-Alkyl oder durch s Reste aus der Gruppe bestehend aus Chlor, Fluor, Methoxy und Ethoxy substituiertes Phenyl,
- s: bedeutet 1, 2 oder 3.

Bevorzugt bedeuten R¹ und R² in Formel (V) unabhängig voneinander Methyl, Ethyl, n-Propyl, iso-Propyl oder n-Butyl Gegenstand der Erfindung sind Verbindungen der Formel (V) worin R¹ für Methyl, und R² für Methyl oder Ethyl steht.

Die nachfolgenden Beispiele erläutern die Erfindung näher. Herstellung von 2-Methyl-3-(methylsulfonyl)-4-(trifluormethyl)benzoesäure Schritt 1: 3-Chlor-2-methyl-4-(trifluormethyl)benzonitril (Variante 1) Unter einer Argonatmosphäre werden 0,44 g NiBr₂ (2 mmol, 2 mol%) und 2,15 g DPEPhos (4 mmol, 4 mol%) mit 0,39 g Zinkpulver (6 mmol, 6 mol%) vorgelegt. Es werden 200 ml Acetonitril zugegeben und die Innentemperatur wird unter Rühren auf 50 °C erhöht. Nach 44 min wird die Innentemperatur auf 10 °C abgesenkt. Nach 48 Minuten bei dieser Temperatur wurde 2,4-Dichlor-3-methyl-trifluormethylbenzol zugegeben (22,9 g, 100 mmol, 1 equiv) gefolgt von Natriumcyanid (5,4 g, 110 mmol, 1.1 equiv). Die Innentemperatur wird auf 80 °C erhöht und die Reaktionsmischung während 18 h kräftig gerührt. Nach Abkühlen wird das Reaktionsgemisch unter reduziertem Druck eingeengt und anschließend mit Heptan und stark verdünnter NaOH versetzt. Das zweiphasige Gemisch wird filtriert, die organische Phase abgetrennt und die wässrige Phase einmal mit Heptan gewaschen. Die vereinten organischen Phasen werden einmal mit Wasser gewaschen und anschliessend unter reduziertem Druck vollständig eingeengt. Man erhält 3-Chlor-2-methyl-4-(trifluormethyl)benzonitril in einer Ausbeute von 90%.
GC/MS: m/e = 219,1 (M); 199,1 (M-HF); 184,1 (M-Cl).

### Schritt 1: 3-Chlor-2-methyl-4-(trifluormethyl)benzonitril (Variante 2)

Unter einer Argonatmosphäre werden 2,96 g NiBr₂-DPEPhos Komplex (3,9 mmol, 1 mol%) und 2,11 g DPEPhos (3,0 mmol, 1 mol%) mit 1,04 g Zinkpulver (15,7 mmol, 4 mol%) vorgelegt. Es werden 400 ml Acetonitril zugegeben und die Innentemperatur wird unter Rühren auf 50 °C erhöht. Nach 20 min wird die Innentemperatur auf 25 °C abgesenkt und es wird 2,4-Dichlor-3-methyl-trifluormethylbenzol (91,6 g, 391,6 mmol, 1 equiv) gefolgt von Natriumcyanid (23,03 g, 469,9 mmol, 1.2 equiv), Zink (0,53 g, 7,8 mmol, 2 mol%) und Wasser (0,16 ml, 8,9 mmol, 2,3 mol%) zugegeben. Die Innentemperatur wird auf 80 °C erhöht und die Reaktionsmischung während 7 h kräftig gerührt. Dann wird erneut Zink (0,53 g, 7,8 mmol, 2 mol%) zugegeben und weitere 13 h kräftig gerührt. Nach Abkühlen wird das Reaktionsgemisch unter reduziertem Druck eingeengt und anschließend mit Methylcyclohexan und stark verdünnter NaOH versetzt. Das zweiphasige Gemisch wird filtriert und die organische Phase abgetrennt. Diese wird mit Wasser gewaschen, über Natriumsulfat getrocknet und anschliessend unter reduziertem Druck vollständig eingeengt. Man erhält 3-Chlor-2-methyl-4-(trifluormethyl)benzonitril in einer Ausbeute von 89%.

### Schritt 2: 2-Methyl-3-(methylsulfanyl)-4-(trifluormethyl)benzonitril

21,4 g 3-Chlor-2-methyl-4-(trifluormethyl)benzonitril (88,7 mmol, 1 equiv) werden bei 50 °C vorlegt. Eine 21%-ige Lösung von NaSMe in H₂O (38,5 ml, 115,4 mmol, 1,3 equiv) wird zugesetzt, gefolgt von Tributyltetradecyl-phosphoniumchlorid (773 mg, 1,78 mmol, 2 mol%). Es wird bei 50 bis 53 °C für 16,5 h gerührt, woraufhin die Reaktionskontrolle per GC vollständigen Umsatz zum Zielprodukt anzeigt. Der Ansatz wird abgekühlt und mit einem Gemisch von MTBE und Wasser versetzt. Die Phasen werden getrennt, die organische Phase zweimal mit Wasser und einmal mit gesättigter wässriger Natriumchloridlösung gewaschen. Dann wird die organische Phase vollständig unter reduziertem Druck eingeengt. Man erhält 23,7 g 2-Methyl-3-(methylsulfanyl)-4-(trifluormethyl)benzonitril (Reinheit laut quant. NMR: 88%, quantitative Ausbeute).

### Schritt 3: 2-Methyl-3-(methylsulfanyl)-4-(trifluormethyl)benzoesäure

Zu 23,4 g 2-Methyl-3-(methylsulfanyl)-4-(trifluormethyl)benzonitril (Reinheit: 90%, 91 mmol, 1 equiv) werden 12,75 g (318,8 mmol, 3,5 equiv) festes NaOH, 12,75 ml Wasser und 105 ml n-Butanol gegeben. Die Mischung wird in einem Ölbad mit 125° Badtemperatur für 5 h erhitzt, woraufhin die Reaktionskontrolle per RP-HPLC vollständigen Umsatz anzeigt. Die Mischung wird auf Raumtemperatur abgekühlt, mit Wasser versetzt, und das Lösungsmittelgemisch wird unter reduziertem Druck entfernt. Es wird nochmals mit Wasser verdünnt und eingeengt um das n-Butanol möglichst vollständig zu entfernen. Anschließend wird etwas Wasser zugesetzt und unter Kühlung mit konzentrierter wässriger HCl angesäuert. Der entstandene schmierige Feststoff wird unter Rühren mit der Zeit fester. Das Gemisch wird im Eisbad abgekühlt, gefiltert und mit kaltem Wasser nachgewaschen. Der Filterkuchen wird zweimal mit 50 ml Heptan gewaschen und anschließend getrocknet. Es werden 23,1 g 2-Methyl-3-(methylsulfanyl)-4-(trifluormethyl)benzoesäure (Reinheit laut quant. NMR: 89%, 90% Ausbeute) als weißer Feststoff erhalten.

### Schritt 4: 2-Methyl-3-(methylsulfonyl)-4-(trifluormethyl)benzoesäure (Variante 1)

2-Methyl-3-(methylsulfanyl)-4-(trifluormethyl)benzoesäure (9,6 g, 38 mmol, 1 equiv) werden in 60 ml Essigsäure-n-butylester gelöst und 1,1 g (3,8 mmol, 0.1 equiv) Natriumwolframat-dihydrat zugegeben. Der Ansatz wird intensiv gerührt und auf 55° C erhitzt. Per Spritzenpumpe werden in 2 Stunden 16,2 ml (190 mmol, 5 equiv) 35%ige Wasserstoffperoxidlösung bei 55-60° Innentemperatur zudosiert. Das Gemisch wird 8 bis 10 Stunden bei dieser Temperatur weitergerührt. Danach wird der Ansatz abgekühlt und mit verd. HCl auf pH = 0 gestellt. Die Reaktionslösung wird auf 60 °C erwärmt, die Phasen werden warm getrennt. Der Großteil des Essigsäure-n-butylesters wird unter reduziertem Druck entfernt. Der entstehende dicke Brei wird abgekühlt und mit wenig Toluol versetzt. Der Niederschlag wird abgesaugt, mit Wasser gewaschen und getrocknet. Man erhält 8,7 g 2-Methyl-3-(methylsulfonyl)-4-(trifluormethyl)benzoesäure (81% Ausbeute) als weißer Feststoff.

### Schritt 4: 2-Methyl-3-(methylsulfonyl)-4-(trifluormethyl)benzoesäure (Variante 2)

2-Methyl-3-(methylsulfanyl)-4-(trifluormethyl)benzoesäure (112,5 g, 450 mmol, 1 equiv) werden in 225 ml Wasser und 56 ml Essigsäure suspendiert. Dann werden 11 g (33,7 mmol) Natriumwolframat-dihydrat zugegeben. Der Ansatz wird intensiv gerührt und auf 95° C erhitzt. Per Spritzenpumpe werden in 4 Stunden 194 g (1,575 mol, 3,5 equiv) 27,6%ige Wasserstoffperoxidlösung bei 95° Innentemperatur zudosiert. Das Gemisch wird 6 Stunden bei dieser Temperatur weitergerührt. Danach wird der Ansatz auf 2 °C abgekühlt, abfiltriert, mit Wasser gewaschen und getrocknet. Man erhält 120 g 2-Methyl-3-(methylsulfonyl)-4-(trifluormethyl)benzoesäure (95% Ausbeute) als weißer Feststoff.

### Ausführungsbeispiele zur Herstellung von Verbindungen der Formel (II)

### Beispiel 1: Herstellung von 2,4-Dichlor-3-methylbenzotrifluorid

Zu einer Lösung aus 2,4-Dichlorbenzotrifluorid (107 g, 0,5 Mol) und Dimethylsulfat (75,6 g, 0,6 mol) in 500 ml THF wurde eine 1 molare Lösung von Lithiumdiisopropylamid in THF (0,6 Mol) bei -50°C langsam zugetropft. Das Gemisch wurde 2 Stunden bei -50 °C gerührt und auf 20 °C erwärmt. Dann wurden 200 ml 1 N HCl als wässrige Lösung langsam zugetropft und danach das THF im Vakuum bei 40°C entfernt. Das Produkt wurde mit 300 ml Hexan extrahiert, das Extrakt mit Wasser gewaschen und über MgSO₄ getrocknet. Das Hexan wurde im Vakuum von 40 mbar entfernt. Man erhielt 110 g eines Gemisches, das nach GC/MS-Analytik 78% 2,4-Dichlor-3-methylbenzotrifluorid, 10% 2,4-Dichlorbenzotrifluorid (Edukt) und 12 % 2,4-Dichlor-3-ethylbenzotrifluorid enthielt. Das gewünschte Produkt kann durch Kristallisation aus Methanol bei -30°C rein erhalten werden.
Ausbeute 73 g (64 % der Theorie). Schmp. 30-32°C. Sdp. 82-84 °C/8 mbar.

### Beispiel 2: Herstellung von 2,4-Dichlor-3-ethylbenzotrifluorid (Variante 1) 2,4-Dichlor-3-ethylbenzotrifluorid

Zu einer Lösung von 2,4-Dichlorbenzotrifluorid (107 g, 0,5 Mol) und Dimethylsulfat (138 g, 1,1 Mol) in 500 ml THF wurde eine 1 molare Lösung von Lithiumdiisopropylamid in THF (1,1 Mol) bei -50 °C langsam zugetropft. Das Gemisch wurde 2 Stunden bei -50°C gerührt und auf 20 °C erwärmt. Dann wurden 100 ml 1 N HCl als wässrige Lösung langsam zugetropft und danach THF im Vakuum bei 40°C entfernt. Das Produkt wurde mit 300 ml Hexan extrahiert, das Extrakt mit Wasser gewaschen und über MgSO₄ getrocknet. Die Lösung wurde im Vakuum bei 40 mbar aufkonzentriert. Man erhielt 125 g eines Gemisches, das nach GC/MS-Analytik 25% 2,4-Dichlor-3-methylbenzotrifluorid, 5% 2,4-Dichlorbenzotrifluorid (Edukt) und 70 % 2,4-Dichlor-3-ethylbenzotrifluorid enthielt. Das gewünschte Produkt kann durch Destillation an einer Vigreux-Kolonne im Vakuum gereinigt werden.
Ausbeute 69 g (58 % der Theorie). Sdp. 114-118°C/15 mbar.

### Herstellung von 2,4-Dichlor-3-methylbenzotrifluorid (Variante 2)

Zu einer Lösung von 2,4-Dichlorbenzotrifluorid (107 g, 0,5 Mol) und Diethylsulfat (92,4 g, 0,6 mol) in 500 ml THF wurde eine 1 molare Lösung von Lithiumdiisopropylamid in THF (0,6 Mol) bei -50 °C langsam zugetropft. Das Gemisch wurde 2 Stunden bei -50°C gerührt und auf 20 °C erwärmt. Dann wurden 100 ml 1 N HCl als wässrige Lösung langsam zugetropft und danach THF im Vakuum bei 40°C entfernt. Das Produkt wurde mit 300 ml Hexan extrahiert, das Extrakt mit Wasser gewaschen und über MgS04 getrocknet. Die Lösung wurde im Vakuum bei 40 mbar aufkonzentriert. Man erhielt 116 g eines Gemisches, das nach GC/MS-Analytik 78% 2,4-Dichlor-3-ethylbenzotrifluorid enthielt. Das gewünschte Produkt kann durch Destillation an einer Vigreux-Kolonne im Vakuum gereinigt werden.

## Patentansprüche

1. Verfahren zur Herstellung von 2-Alkyl-4-trifluormethyl-3-alkylsulfonylbenzoesäuren der allgemeinen Formel (I),
**dadurch gekennzeichnet, dass**
a) in einem ersten Schritt ein 1,3-Dichlor-2-alkyl-4-trifluormethylbenzol mit einer Cyanidquelle in Gegenwart einer Nickelverbindung, eines Phosphinliganden und eines weiteren Metalles zu einem Benzonitril umgesetzt wird,
b) in einem zweiten Schritt das Benzonitril mit einem Thiolat in Anwesenheit eines Phasentransferkatalysators zum entsprechenden Thioether umgesetzt wird,
c) in einem dritten Schritt die Nitrilgruppe zur Carboxylgruppe verseift wird,
d) in einem vierten Schritt die Thiogruppe oxidiert wird, gegebenenfalls in Gegenwart eines Oxidationskatalysators, 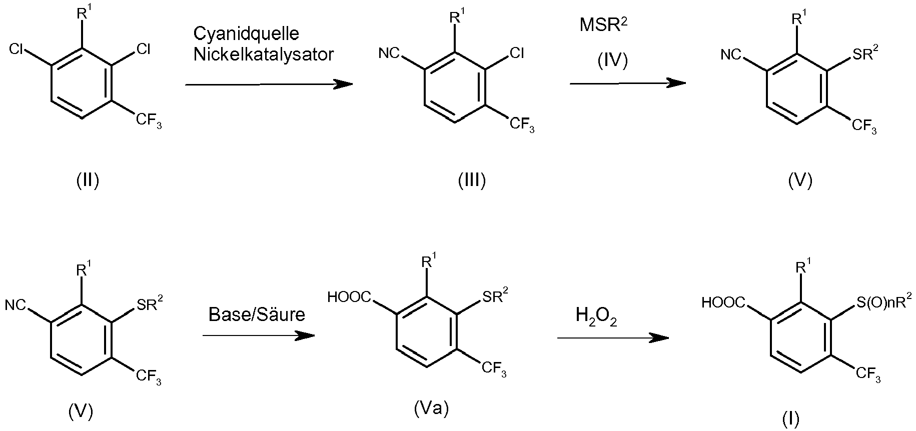 und
e) worin die Substituenten wie nachfolgend definiert sind:
R¹ und R² bedeuten unabhängig voneinander C₁-C₄-Alkyl oder durch s Reste aus der Gruppe bestehend aus Chlor, Fluor, Methoxy und Ethoxy substituiertes Phenyl,
M bedeutet Lithium, Natrium oder Kalium,
n bedeutet 1 oder 2,
s bedeutet 1, 2 oder 3.

2. Verfahren nach Anspruch 1, worin R¹ und R² jeweils für Methyl stehen.

3. Verfahren nach Anspruch 1 oder 2, worin als Cyanidquelle Natriumcyanid, Kaliumcyanid, Zinkcyanid, Kaliumhexacyanoferrat(II) oder Acetoncyanhydrin verwendet wird.

4. Verfahren nach einem der Ansprüche 1 bis 3, worin die Cyanidquelle in einem Molverhältnis von 1:1 bis 1,5:1, bezogen auf die Verbindung der allgemeinen Formel (II) eingesetzt wird.

5. Verfahren nach einem der Ansprüche 1 bis 4, worin für die Nickelkatalysator Herstellung Nickel(II)chlorid oder Nickel(II)bromid in einer Menge von 0,5 bis 5 Molprozent, und der Phosphinligand in einer Menge von 0,5 bis 10 Molprozent bezogen auf die Verbindung der allgemeinen Formel (II) eingesetzt wird.

6. Verfahren nach einem der Ansprüche 1 bis 5, worin als Phosphinligand Bis(2-diphenylphosphinophenyl)ether (dpephos), Bis(diphenylphosphino)ferrocen (dppf) oder rac-2,2'-Bis(diphenylphosphino)-1,1'-binaphthyl (BINAP) in einem Molverhältnis von 1,5:1 bis 3:1, bezogen auf die Nickelverbindung, eingesetzt wird.

7. Verfahren nach einem der Ansprüche 1 bis 6, worin als Phosphinligand Bis(2-diphenylphosphinophenyl)ether (dpephos) eingesetzt wird.

8. Verfahren nach einem der Ansprüche 1 bis 7, worin als Reduktionsmittel 2 bis 10 Molprozent Zink, bezogen auf die Verbindung der Formel (II), eingesetzt wird.

9. Verfahren nach einem der Ansprüche 1 bis 8, worin als Oxidationskatalysator Na₂WO₄ in einer Menge von 5 bis 15 Molprozent, und Wasserstoffperoxid in einer Menge von 3 bis 8 Moläquivalenten, jeweils bezogen auf die Verbindung der allgemeinen Formel (Va), eingesetzt werden.

10. Verbindungen der Formel (IIa): 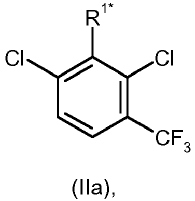 worin R^{1*} für Ethyl, n-Propyl oder Phenyl steht.

11. Verbindung der Formel (IIIa), 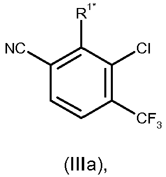 worin R^{1*} für Methyl steht.

12. Verbindungen der Formel (V), 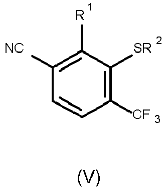 worin
R¹ für Methyl steht, und
R² für Methyl oder Ethyl steht.

## Claims

1. Process for preparing 2-alkyl-4-trifluoromethyl-3-alkylsulfonylbenzoic acids of the general formula (I), **characterized in that**
a) in a first step, a 1,3-dichloro-2-alkyl-4-trifluoromethylbenzene is reacted with a cyanide source in the presence of a nickel compound, a phosphine ligand and a further metal to form a benzonitrile,
b) in a second step, the benzonitrile is reacted with a thiolate in the presence of a phase transfer catalyst to form the corresponding thioether,
c) in a third step, the nitrile group is hydrolyzed to a carboxyl group,
d) in a fourth step, the thio group is oxidized, optionally in the presence of an oxidation catalyst, 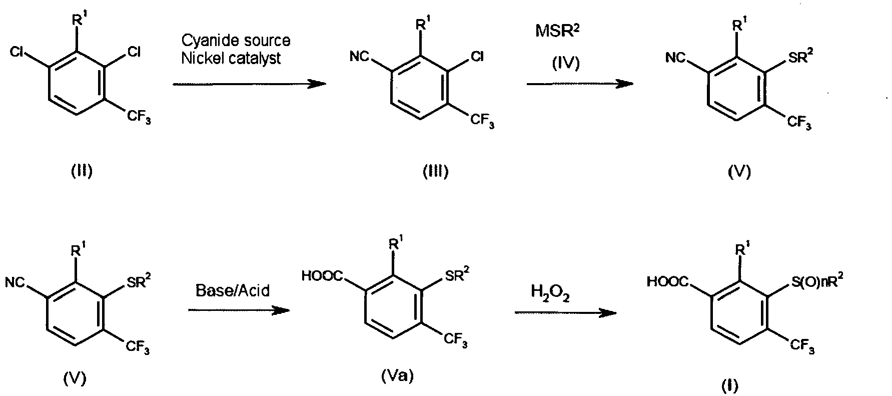 and
e) where the substituents are defined as follows:
R¹ and R² are each, independently of one another, C₁-C₄-alkyl or phenyl substituted by s radicals from the group consisting of chloro, fluoro, methoxy and ethoxy,
M is lithium, sodium or potassium,
n is 1 or 2,
s is 1, 2 or 3.

2. Process according to Claim 1, wherein R¹ and R² are each methyl.

3. Process according to Claim 1 or 2, wherein sodium cyanide, potassium cyanide, zinc cyanide, potassium hexacyanoferrate (II) or acetone cyanohydrin is used as cyanide source.

4. Process according to any of Claims 1 to 3, wherein the cyanide source is used in a molar ratio of from 1:1 to 1.5:1, based on the compound of the general formula (II) .

5. Process according to any of Claims 1 to 4, wherein nickel(II) chloride or nickel(II) bromide in an amount of from 0.5 to 5 mol percent and the phosphine ligand in an amount of from 0.5 to 10 mol percent based on the compound of the general formula (II) are used for producing the nickel catalyst.

6. Process according to any of Claims 1 to 5, wherein bis(2-diphenylphosphinophenyl) ether (dpephos), bis(diphenylphosphino)ferrocene (dppf) or rac-2,2'-bis(diphenylphosphino)-1,1'-binaphthyl (BINAP) is used as phosphine ligand in a molar ratio of from 1.5:1 to 3:1, based on the nickel compound.

7. Process according to any of Claims 1 to 6, wherein bis(2-diphenylphosphinophenyl) ether (dpephos) is used as phosphine ligand.

8. Process according to any of Claims 1 to 7, wherein from 2 to 10 mol percent of zinc, based on the compound of the formula (II), are used as reducing agent.

9. Process according to any of Claims 1 to 8, wherein Na₂WO₄ is used in an amount of from 5 to 15 mol percent as oxidation catalyst and hydrogen peroxide is used in an amount of from 3 to 8 molar equivalents, in each case based on the compound of the general formula (Va).

10. Compound of the formula (IIa): 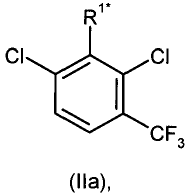 wherein R^{1*} is ethyl, n-propyl or phenyl.

11. Compound of the formula (IIIa): 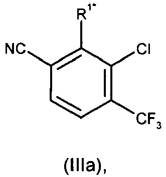 wherein R^{1*} is methyl.

12. Compound of the formula (V), 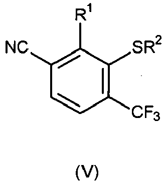 wherein
R¹ is methyl and
R² is methyl or ethyl.

## Revendications

1. Procédé pour la préparation d'acides 2-alkyl-4-trifluorométhyl-3-alkylsulfonylbenzoïques de formule générale (I),
**caractérisé en ce que**
a) dans une première étape, un 1,3-dichloro-2-alkyl-4-trifluorométhylbenzène est transformé avec une source de cyanure en présence d'un composé de nickel, d'un ligand phosphine et d'un autre métal, en un benzonitrile,
b) dans une deuxième étape, le benzonitrile est transformé avec un thiolate en présence d'un catalyseur de transfert de phase, en thioéther correspondant,
c) dans une troisième étape le groupe nitrile est saponifié en groupe carboxyle,
d) dans une quatrième étape, le groupe thio est oxydé, éventuellement en présence d'un catalyseur d'oxydation, 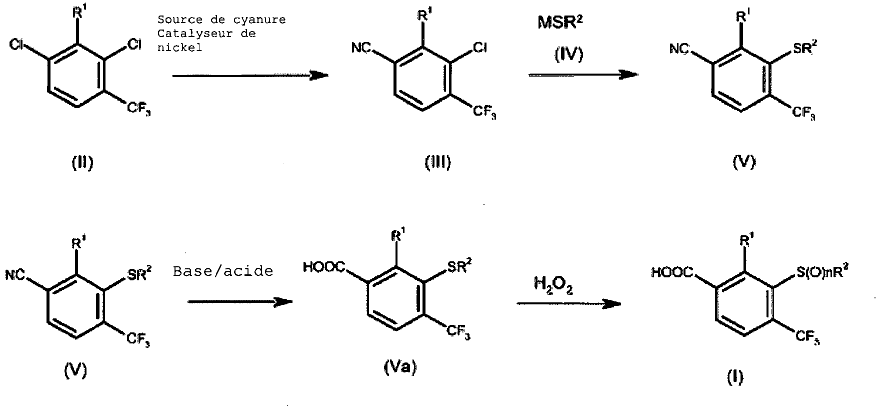 et
e) dans lesquelles les substituants sont définis comme suit :
R¹ et R² signifient indépendamment l'un de l'autre C₁₋₄-alkyle ou phényle substitué par s radicaux du groupe constitué par chlore, fluor, méthoxy et éthoxy,
M signifiant lithium, sodium ou potassium,
n signifiant 1 ou 2,
s signifiant 1, 2 ou 3.

2. Procédé selon la revendication 1, R¹ et R² représentant à chaque fois méthyle.

3. Procédé selon la revendication 1 ou 2, du cyanure de sodium, du cyanure de potassium, du cyanure de zinc, de l'hexacyanoferrate (II) de potassium ou de la cyanhydrine d'acétone étant utilisé(e) en tant que source de cyanure.

4. Procédé selon l'une quelconque des revendications 1 à 3, la source de cyanure étant utilisée en un rapport molaire de 1:1 à 1,5:1, par rapport au composé de formule générale (II).

5. Procédé selon l'une quelconque des revendications 1 à 4, dans lequel, pour la préparation du catalyseur de nickel, du chlorure de nickel (II) ou du bromure de nickel (II) est utilisé en une quantité de 0,5 à 5 pour cent en moles, et le ligand phosphine est utilisé en une quantité de 0,5 à 10 pour cent en moles par rapport au composé de formule générale (II).

6. Procédé selon l'une quelconque des revendications 1 à 5, dans lequel le bis(2-diphénylphosphinophényl)éther (dpephos), le bis(diphénylphosphino)ferrocène (dppf) ou le rac-2,2'-bis(diphénylphosphino)-1,1'-binaphtyle (BINAP) est utilisé en tant que ligand phosphine en un rapport molaire de 1,5:1 à 3:1, par rapport au composé de nickel.

7. Procédé selon l'une quelconque des revendications 1 à 6, dans lequel le bis(2-diphénylphosphinophényl)éther (dpephos) est utilisé en tant que ligand phosphine.

8. Procédé selon l'une quelconque des revendications 1 à 7, dans lequel 2 à 10 pour cent en moles de zinc, par rapport au composé de formule (II), est utilisé en tant que qu'agent de réduction.

9. Procédé selon l'une quelconque des revendications 1 à 8, dans lequel Na₂WO₄, en une quantité de 5 à 15 pour cent en moles, et du peroxyde d'hydrogène en une quantité de 3 à 8 équivalents en moles, à chaque fois par rapport au composé de formule générale (Va), sont utilisés en tant que catalyseur d'oxydation.

10. Composés de formule (IIa) : 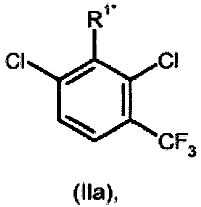 dans laquelle R^{1*} représente éthyle, n-propyle ou phényle.

11. Composé de formule (IIIa) : 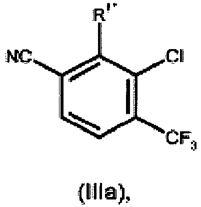 dans laquelle R^{1*} représente méthyle.

12. Composés de formule (V), 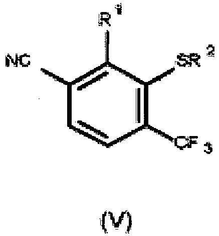 dans laquelle
R¹ représente méthyle, et
R² représente méthyle ou éthyle.
